# EUROPEAN PATENT APPLICATION

(11) **EP 1 118 661 A1**
(43) Date of publication of application: **25.07.2001**
(21) Application number: 00200110.5
(22) Date of filing: 13.01.2000
(51) Int. Cl.: C12N 15/10, C12N 15/86, C07K 14/705, C12Q 1/68, G01N 33/566

(54) **T cell receptor libraries**

(71) Applicant: Het Nederlands Kanker Instituut, 1066 CX Amsterdam (NL)
(72) Inventor: SCHUMACHER, Antonius Nicolaas Maria, 2024 DL Haarlem (NL); KESSELS, Helmut Wilhelminus Hubertus Gerardus, 1073 VX Amsterdam (NL)
(74) Representative: Ottevangers, Sietse Ulbe

(57) **Abstract**

The invention is concerned with the generation of receptors with a desired specificity for a ligand, wherein the receptor upon binding to the ligand undergoes functional processing in order to provide a biological response after ligand binding.

## Description

The invention relates to the field of molecular biology, in particular molecular biology related to specific receptor-ligand interactions, more in particular immunology and novel approaches of treating diseases somehow associated with receptor ligand interaction, more in particular an immune response or its absence. The acquired immune system (in mammalians) comprises two major kinds of responses; the so-called humoral response involving antibodies and the so-called cellular response involving T cells.

T cells, the prime mediators of adaptive cellular immunity, specify their action through the TCR-mediated recognition of a peptide epitope bound to a major histocompatibility complex (MHC) molecule. The immune system contains a large collection of T cells that covers a broad range of peptide/MHC specificities and thereby can identify subtle changes in MHC epitope presentation. However, negative and positive selection processes in the thymus impose a restriction on T cell diversity and thereby limit the spectrum of *in vivo* T cell reactivity. For instance, self-tolerance leads to the removal of the high affinity T cell repertoire specific for self antigens, and this will include T cells with desirable specificities, such as for self antigens expressed on tumor tissues ^{1,2}. Because of the potential value of an experimental approach that can be used to isolate T cell receptors with desirable specificities we set out to develop a strategy for in vitro TCR selection.

For the *in vitro* isolation and generation of monoclonal antibodies, antibody phage display has proven to be a useful technology to replace hybridoma technology and animal immunization ³. Recently, the expression of single-chain TCRs by filamentous phages has likewise been achieved 4 and this approach may conceivably be used to produce phage display libraries for TCR selection purposes. However, the ability of T cell membrane-associated TCRs to discriminate between closely related ligands appears to be directly related to the property of TCRs to cluster upon encounter of their cognate ligands ^{5,6}, and it may prove difficult to copy this process on phage. The present invention in one embodiment therefore provides a strategy for TCR-display that closely mimics the *in vivo* situation, exemplified by retroviral insertion of a T cell receptor library into a TCR-negative T cell host. Such T cell line-displayed TCR libraries would not only allow the selection of desirable TCRs by biochemical means, but also offer the possibility to directly test the functional behavior of selected TCRs.

This approach however may not be limited to T cell receptors alone. Other receptors that need to undergo a functional reorganisation, such as a conformation change, binding to other proteinaceous substances, clustering and/or internalisation may also be treated and developed in accordance with the present invention.

Thus the invention provides a method for generating at least one receptor having a desired specificity for a ligand, whereby said receptor undergoes functional processing in order to provide a biological response (thus the receptor should have this capability) after ligand-binding, comprising constructing a sequence encoding such a receptor and allowing for the product of said sequence to be expressed in a suitable environment wherein said processing after ligand binding can occur. A receptor according to the invention may be a recombinantly produced natural receptor or a mutated receptor in which any (binding) characteristic has been altered. A ligand for such a receptor may range from a steroid (a small organic molecule) to a proteinaceous substance, including preferred ligands such as peptides. As stated herein before, the receptor can according to the invention be functionally tested in its environment because the environment allows for the normal fucntional changes that such a receptor undergoes upon binding of its ligand. A preferred group of receptors typically often undergoing such processing are membrane associated receptors.

These include transmembrane receptors such as T cell receptors, immunoglobulins, NK cell receptors, olfactory receptors

The suitable environment for these kind of receptors of course includes a membrane-like structure, such as a liposome, a microsome, or a cell, of which the last one is preferred. The last one is preferred, because a cell may provide other components of a suitable environment, such as signalling pathways and the like.

As stated herein before in a preferred embodiment the invention provides a method for generating at least one receptor having a desired specificity for a ligand, whereby said receptor undergoes functional processing after ligand-binding, comprising constructing a sequence encoding such a receptor and allowing for the product of said sequence to be expressed in a suitable environment wherein said processing after ligand binding can occur, wherein said receptor is a T cell receptor. T cell receptors, as will be explained in the detailed description below are available or produced by mammalians in many specificities, typically excluding specificities of the mammalian itself. It is in these specificities that one of the main interests of the present invention lies. These specificities may be the ones that can be used to fight tumours in e.g. a gene therapy setting, whereby a patient's T cells are provided with a receptor produced according to the invention. These preferred T cell receptors can be produced advantageously in T cells lacking T cell receptors themselves, since this is probably the most suitable environment for their post-binding processing. Thus in a preferred embodiment the invention provides a method for generating at least one T cell receptor having a desired specificity for a ligand, whereby said receptor undergoes functional processing after ligand-binding, comprising constructing a sequence encoding such a receptor and allowing for the product of said sequence to be expressed in a suitable host cell wherein said processing after ligand binding can occur, wherein said host cell is a T cell receptor negative T cell. In some instances it may be preferred to have the sequence encoding the produced, optionally mutated, receptor into the genome of the host cell. This can be advantageously achieved by providing the sequence encoding the receptor in a retroviral delivery vehicle. The host cell can then be simply infected with a retrovirus provided with such an extra sequence.

Retroviruses capable of infecting e.g. T cells are known in the art and need no further elaboration here.

The present invention is typically also aimed at providing libraries of receptors having all kinds of different known and/or unknown binding affinities for ligands. In order to produce such different affinities natural receptor encoding sequences may be modified by any known means, such as site-directed mutation, genetic drift, shuffling, etc. All these methods will lead to a modified receptor encoding sequence, which for the sake of simplicity will be referred to as sequences comprising mutations. Specifically any modified receptor encoding sequence in this invention may be referred to as a mutated constructed sequence. Typically such mutations are directed at modifying the binding affinity of the receptor for a ligand, in the case of e.g. T cell receptors the affinity may be changed to an affinity normally suppressed in said receptor's natural surrounding. It is clear that such affinities are of use in treating diseases such as cancer. Thus in a preferred embodiment the invention provides a method wherein said receptor is a T cell receptor having affinity for a self antigen, a tumour antigen and/or a synthetic antigen.

As stated before a main goal of the present invention is to arrive at libraries of e.g. cells having receptors of many different affinities. Thus in a further preferred embodiment the invention provides a method as disclosed before wherein a number of different constructed sequences are brought into separate suitable environments providing a library of environments having receptors with different ligand binding affinities. Preferred libraries of receptors according to the invention are libraries wherein said receptors are T cell receptors. The libraries are of course used to identify T cell receptors or other receptors which have affinity for a desired ligand. The genetic information encoding such a receptor may then be taken from its environment and expressed in any desired environment. The original suitable environment may of course also be used. Thus the invention also provides a method for selecting a T cell receptor or a sequence encoding the same, comprising contacting a ligand to be recognised by said T cell receptor with a library according to the invention in the appropriate context and selecting at least one binding T cell receptor from said library. The ligand must of course be offered to the receptor in a suitable context. For T cell receptors this would mean that a peptide must be presented in the right MHC context.

T cell receptors and their encoding sequences identified and obtained according to any method of the invention are of course also part of the present invention. As an example these T cell receptors or typically the encoding sequence can be brought into a T cell of a host in order to provide such a host with additional capability of attacking e.g. a tumour. Thus the invention also provides a method for providing a T cell with the capability of binding a desired presented antigen, comprising providing said T cell with a T cell receptor or a sequence encoding it according to the invention. The resulting T cell is again part of the invention.

This T cell can be reintroduced into a patient. Thus the invention further provides a method for providing a subject with additional capability of generating a response against antigens of undesired cells or pathogens, comprising providing said subject with at least one T cell according to the invention. Preferably said T cell is derived from said subject, at least it should be HLA matched for said subject.

### Detailed description.

For the development of TCR display two recent breakthroughs have been extremely valuable. The elucidation of the structure of human and mouse class I MHC-TCR complexes ^{7,8} allows the design of TCR libraries that selectively target the peptide specificity of such receptors. In addition, the development of MHC tetramer technology 9 has provided the means with which to isolate cells carrying variant receptors, solely on the basis of their antigen specificity.

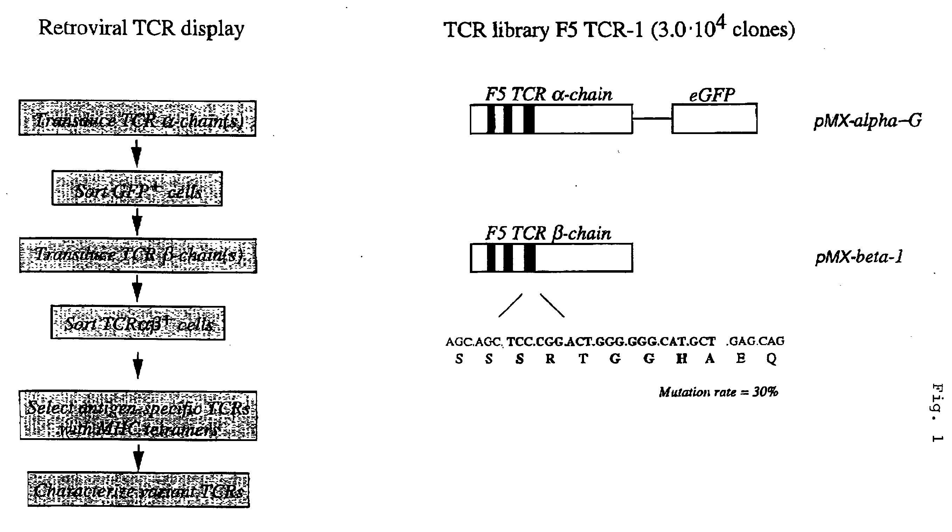

Through the generation and screening of an *in vitro* T cell library based on an influenza A-specific T cell receptor (Figure 1), we have isolated variant TCRs that are either specific for the parental viral strain, or that have acquired a specificity for a variant influenza epitope. These *in vitro* selected T cell receptors recognize peptide-MHC complexes on target cells with high efficiency and high specificity. The ability to control TCR fine specificity in a direct manner by retroviral display provides a general strategy for the generation of T cells with specificities that could previously not be obtained. In addition, retroviral TCR display offers a powerful strategy to dissect structure-function relationships of the T cell receptor in a physiological setting.

We here describe a strategy used to change the ligand specificity of a T cell receptor. By generating a TCR library that is diversified in its CDR3β structure, we were able to select novel TCRs that either share specificity with the parental TCR, or that have acquired a specificity for a variant T cell epitope. A change in TCR specificity can be thought of as an increase in TCR affinity for the variant epitope. The F5 TCR does not measurably bind to the A/PR8/34 epitope, but we were able to transform it into a high affinity TCR for this antigen. The possibility to change a low affinity, non-functional receptor into a highly potent TCR by retroviral display is useful for the creation of collections of optimized pathogen-specific T cell receptors. In addition, this *in vitro* strategy is particularly valuable for the development of high affinity tumor-specific T cell receptors. In a number of systems it has been demonstrated that self-tolerance results in the removal of the high avidity T cell repertoire specific for tumor-lineage antigens ²²⁻²⁵. The deletion of self-specific T cells does not affect T cells that have a low affinity for these antigens ²⁶⁻²⁹ (de Visser *et al*, ms. submitted). However, it has become clear that these low affinity T cells only display anti-tumor activity in those special cases in which the self-antigen is overexpressed in the tumor tissue ²⁷, and are ineffective in most cases ^{28,30}. The retroviral TCR display system outlined here provides a unique opportunity to convert low affinity receptors into high affinity tumor-lineage-specific TCRs, and the creation of a collection of high affinity T cell receptors that target lineage antigens expressed on tumor tissues is thereby now feasible.

### Creation of a retroviral TCR display library

As a host for a T cell line-displayed TCR library, an immature T cell line that does not express endogenous T cell receptor α and β chains was selected. This cell line, named 34.1L, expresses all CD3 components required for TCR assembly, but is devoid of CD4 or CD8 co-receptor expression. Because initial experiments indicated that the expression of the CD3ζ TCR component was limiting in this cell line, a variant T cell line (34.1Lζ) was produced in which a CD3ζ encoding vector was introduced by retroviral gene transfer.

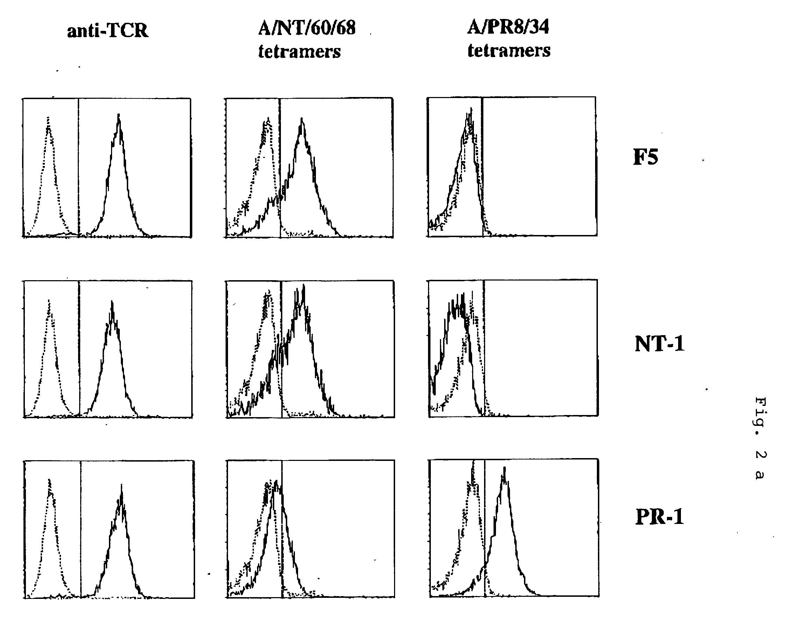

As a model system for the generation of a TCR display library we used a high affinity murine TCR of which the antigen specificity is well established 10. This F5 T cell receptor (Vα4;Vβ11) specifically recognizes the immunodominant H-2D^{b}-restricted CTL epitope NP₃₆₆₋₃₇₄ (ASNENMDAM) of the influenza A/NT/60/68 nucleoprotein 11. Following introduction of the F5 TCR in the 34.1Lζ cell line by retroviral transduction, the transduced cell line expresses high levels of the introduced F5 TCR as measured by anti-TCRβ and MHC tetramer flow cytometry (Figure 2A).

To test the merits of retroviral TCR display for the selection of TCRs with defined specificities, we aimed to isolate novel T cell receptors with either the same specificity as the parental TCR, or receptors that have acquired a specificity for a variant influenza epitope. In order to modify the peptide specificity of TCRs without generating variant TCRs that are broadly cross-reactive, we set out to mutate those areas of the TCR that primarily interact with the antigenic peptide. Structural analysis of four different human and mouse αβTCRs in complex with their cognate peptide/MHC class I all point to the CDR3 loops of the TCRα and β chain as the major determinants of peptide specificity 7,8,12,13. In all cases examined, the TCR binds diagonally across the MHC class I/peptide complex such that the N-terminal part of the MHC-bound peptide is primarily in contact with the TCRα CDR3, whereas the C-terminal part mainly interacts with the CDR3 of the TCRβ chain. Because in the current set of experiments we were primarily interested in obtaining TCRs that can discriminate between epitopes that differ in the C-terminal half of the peptide (see below), a TCR library was manufactured such that its structural diversity is directed towards the TCRβ CDR3 loop exclusively. Through PCR assembly 14 a F5 TCRβ DNA library was generated that contains a 30% mutational rate in its 7 amino acid CDR3 (Figure 1). The 34.1L ζ cell line was transduced with the F5 TCRα DNA and the TCRβ DNA library to generate a library of T cells with variant CDR3β loops, and 3.0x10⁴ surface TCR expressing cells were isolated by flow cytometry. Sequence analysis of single cell clones from TCR expressing cells were used to provide an estimate of the structural requirements for TCR cell surface expression. and the CDR3β sequence was determined. These data indicate that the serine on position 1 in the CDR3 is conserved and that for the glycine pair on positions 4 and 5 only conservative amino acid substitutions (alanine/serine) are allowed for all mutant TCRs that are expressed at the cell surface (data not shown).

### Isolation of variant T cell receptors

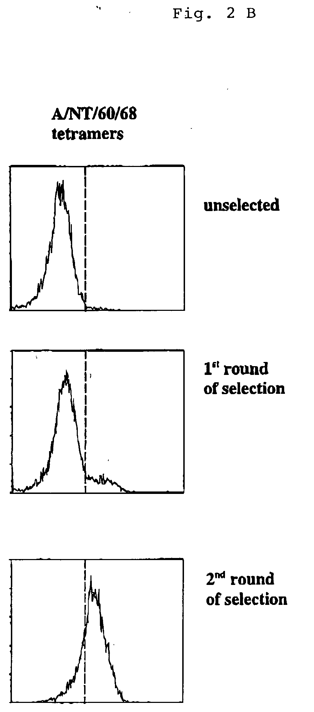

To examine whether variant TCRs could be obtained that retain the ligand specificity of the parental F5 TCR, the T cell library was screened for binding of tetrameric H-2D^{b} complexes containing the A/NT/60/68 nucleoprotein CTL epitope (ASNENMDAM) (Figure 2B). Following a first selection round, a population of H-2D^{b} tetramer reactive cells was isolated by flow cytometry. Sequence analysis of the CDR3β loops within this population reveals that although this population is divers, at most positions within the CDR3β only conservative amino acid mutations are allowed for recognition of the A/NT/60/68 NP₃₆₆₋₃₇₄ tetramers (data not shown). In order to enrich for TCRs with highest affinity for the A/NT/60/68 epitope, a subsequent more stringent selection round was performed in which tetramer-high, TCR-low cells were isolated. In this population two different clones persisted: the parental F5 clone and a variant clone named NT-1. The CDR3β DNA sequence of the NT-1 TCR contains five mutations that result in three conservative amino acid substitutions (table 1). This variant TCR appears to bind the A/NT/60/68 NP₃₆₆₋₃₇₄ tetramers with similar efficiency as the F5 TCR (Figure 2A).

The TCRβ CDR3 library was subsequently screened for the presence of T cell receptors that bind H-2D^{b} tetramers containing a variant influenza A nucleoprotein epitope. This variant NP₃₆₆₋₃₇₄ epitope (ASNENMETM), derived from the influenza A/PR8/34 strain, differs from the A/NT/60/68 CTL epitope by two conservative amino acid substitutions in the C-terminal half of the peptide and is not recognized by the F5 T cell receptor ¹⁰ (Figure 2A). The TCRβ CDR3 library was subjected to multiple rounds of selection with H-2D^{b} tetramers that contain the variant epitope, in order to select for the TCR clone(s) that exhibit highest affinity for this epitope. After four selection rounds a single TCR clone emerged (named PR-1) that avidly binds to the A/PR8/34 NP₃₆₆₋ ₃₇₄ tetramers (Figure 2B). Interestingly, although in this library screen we did not select against reactivity with the A/NT/60/68 T cell epitope, the PR-1 TCR appears to have lost the ability to react with H-2D^{b} tetramers that contain this original epitope. Sequence analysis of the PR-1 TCR reveals 7 nucleotide mutations in its CDR3β DNA sequence compared to the parental F5 TCR. These mutations result in 4 conservative amino acid changes and one non-conservative Arg to Trp substitution (Table 1).

### In vitro function of selected T cell receptors

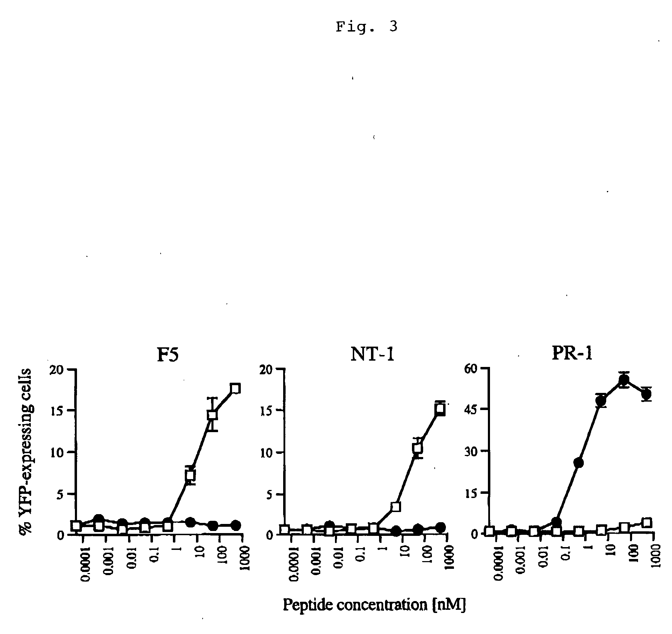

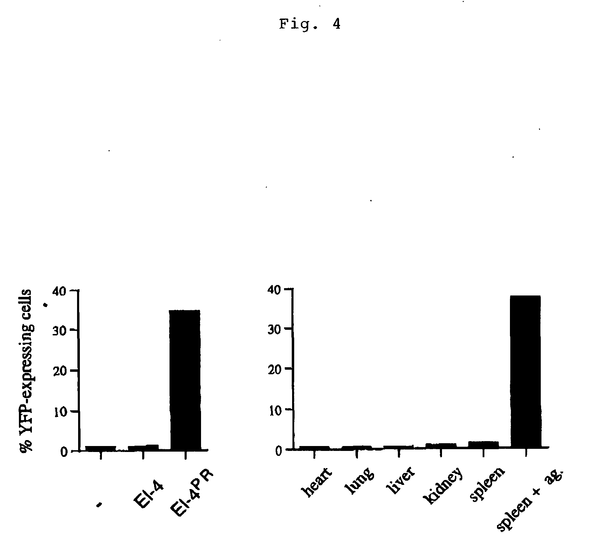

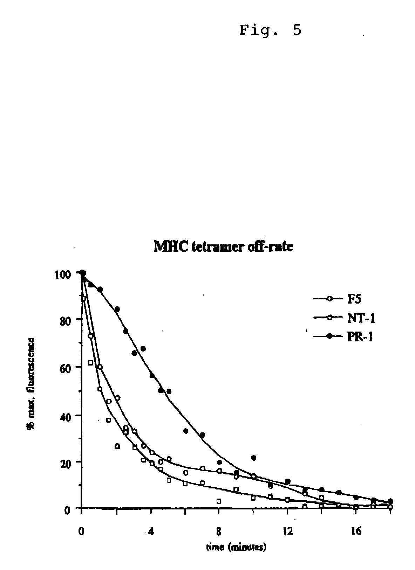

To examine whether *in vitro* selected variant TCRs can evoke T cell activation upon peptide recognition, ligand-induced IL-2 gene transcription was measured. To this purpose we used a self-inactivating (SIN) retroviral vector containing multiple NFAT binding sites upstream of a minimal IL2 promoter and the reporter gene YFP. In T cells that are transfected with this construct, the binding of NFAT transcription factors to the NFAT promotor element offers a direct reflection of T cell activation ^{15,16} (Hooijberg *et al,* ms. submitted) . 34.1Lζ cells expressing the F5, NT-1 or PR-1 TCR were virally transduced with the NFAT-YFP reporter construct and these transduced cells were subsequently exposed to target cells in the presence of different concentrations of either the A/NT/60/68 or A/PR8/34 T cell epitope. Both variant clones NT-1 and PR-1 efficiently induce T cell activation upon specific antigen recognition with an absolute specificity for the epitope used during the *in vitro* selections (Figure 3). Remarkably, the PR-1 TCR shows a greater than ten-fold increased sensitivity for its ligand, as compared to the recognition of the A/NT/60/68 epitope by the F5 TCR. Even though F5 T cells obtained from TCR-transgenic mice are readily activated by low levels of endogenously produced A/NT/60/68 epitopes, both the NT-1 and F5 TCR-transduced 34.1Lζ cells do not efficiently recognize EL4 cells that endogenously produce the A/NT/60/68 CTL epitope, presumably due to the absence of the CD8 co-receptor on this cell line (not shown). In contrast, recognition of endogenously produced A/PR8/34 nucleoprotein epitopes is readily observed for the PR-1 receptor, indicating that this receptor can function in a CD8-independent fashion (Fig. 4, left). This high TCR sensitivity is not a result of an increased TCR cell surface expression (Figure 2B) and may therefore be a direct reflection of a decrease in TCR-MHC off-rate 17-19. To address this issue, MHC-TCR dissociation rates were determined, by measuring the decay of peptide/H-2D^{b}-tetramer staining upon addition of an excess of the homologous H-2D^{b} monomer (Figure 5). The half-life of the PR-1/MHC complex as measured in this assay is approximately 4 fold longer, as compared to that of the F5/MHC complex. In line with the functional data, the off-rate of the NT-1/MHC complex is similar to that of the high affinity F5 TCR.

These experiments reveal that *in vitro* selection of variant T cell receptors by retroviral TCR display can yield receptors with high potency, as revealed by both biochemical means and functional assays. This despite the fact that in the current set of experiments only the CDR3 region of the TCRβ chain was targeted, and that the length of this CDR3 loop was kept constant. In addition, the diversity of the library used in these experiments (3x10⁴ independent clones) was relatively modest. However, we estimate that through optimization of transduction and sorting strategies retroviral TCR display libraries of 10⁶-10⁷ in size are technically achievable in this system. Such *in vitro* TCR libraries will then enclose a diversity of ligand specificities that approaches that of the total human naïve TCR repertoire (2.5x10⁷) 20. Because retroviral TCR libraries can be focussed towards specific antigen recognition as shown here, the isolation of TCRs with desirable specificities from such *in vitro* display systems may in fact be relatively straightforward. The T cell receptors that are isolated in this manner may be used to for the creation of redirected T cell populations, through gene transfer of peripheral T cell populations²¹. To provide a first estimate of the risk of autoreactivity following creation of cells that carry *in vitro* manipulated T cell receptors, PR-1 expressing cells were exposed to an array of different tissue samples from H-2D^{b}- expressing mice. Even though a strong T cell responses is induced by splenocytes that are incubated with the influenza A CTL epitope, no T cell activation above background values is observed upon incubation with a range of self tissues (Figure 4, right).

### Methods

**Preparation of H-2D**^{**b**} **tetramers.** Peptides were produced using standard Fmoc chemistry. Soluble allophycocyanin (APC)-labeled H-2D^{b} tetramers were produced as described previously ^{9,31} and stored frozen in Tris-buffered saline/16% glycerol/0.5% BSA.

### Cell lines and viruses. The 34.1L cell line is a day 14

fetal thymus derived prethymocyte cell line 32 and was a kind gift of Dr. A. Kruisbeek (NCI, Amsterdam, the Netherlands). The Phoenix-A cell line, a derivative of the human embryonic kidney cell line 293T, was a kind gift of Dr. G. Nolan (Stanford University, Palo Alto, CA). The EL4 tumor cell line is a murine thymoma cell line of the H-2^{b} haplotype 33.

The EL4^{PR} cell line was obtained by transduction of EL4 cells with a retrovirus encoding the eGFP gene with the A/PR/8/34 CTL epitope as a C-terminal fusion, and was isolated by fluorescence-activated cell sorting of eGFP-expressing cells (M.C. Wolkers *et al.,* ms in preparation). For the generation of the 34.1Lζ cell line, CD3ζ cDNA was amplified by PCR with primers CD3ζtop (CCCAAGCTTATGAAGTGGAAAGTGTCTGTTC) and CD3ζbottom (ATAAGAATGCGGCCGCTTACTGGTAAAGGCCATCGTG) (Isogen Bioscience BV, Maarssen, the Netherlands), and subcloned into the retroviral vector pMX (a kind gift from Dr. T. Kitamura, University of Tokyo, Japan). Retroviral supernatant was produced in Phoenix-A cells and was used to transduce 34.1L cells. Following transduction, 34.1Lζ cells were cloned and expression of the transduced CD3ζ chain was assessed by RT-PCR. All cell lines were grown in Iscove's modified Dulbecco's medium (Life Technologies BV, Scotland) supplemented with 5% fetal calf serum (BioWhittaker, Belgium), 0.5 mM β-mercaptoethanol (Merck, Darmstadt, Germany), penicillin (100 U/ml) and streptomycin (100 µg/ml) (Boehringer Mannheim, Germany).

**Production of retroviral supernatants and retroviral transduction**. Plasmid DNA was transfected into Phoenix-A cells by pfx-2 lipid transfection (Invitrogen). After transfection the cells were cultured for 48 hours prior to the transduction procedure. The recombinant human fibronectin fragments CH-296 transduction procedure (RetroNectin™; Takara, Otsu, Japan) was based on a method developed by Hanenberg *et al* ³⁴. Non-tissue culture treated Falcon petridishes (3 cm diameter) (Becton Dickinson) were coated with 2 ml of 30 µg/ml recombinant human fibronectin fragment CH-296 at room temperature for 2 hours. The CH-296 solution was removed and replaced with 2 ml 2% bovine serum albumin (Sigma) in PBS for 30 min at room temperature. The target cells were plated on RetroNectin™ coated dishes (0.5x10⁶ cells/petridish) in 1 ml of retroviral supernatant. Cells were cultured at 37°C for 24 hours, washed and transferred to 25 cm² culture flasks (Falcon plastics, Becton Dickinson).

**Construction of the F5 TCR CDR3 library**. TCR cDNAs were generated from F5 TCR transgenic T cells by reverse transcriptase reaction (Boehringer Mannheim, Germany). The F5 TCRα cDNA was amplified by PCR with F5α-top (GGGGGATCCTAAACCATGAACTATTCTCCAGCTTTAGTG) and F5α-bottom (GGAAGGGGGCGGCCGCTCAACTGGACCACAGCCTCAG) primers (Perkin Elmer, Nieuwekerk a/d IJssel, The Netherlands) and ligated into the pMX-IRES-eGFP vector. The F5 TCRβ cDNA was amplified by PCR with F5β-top (GGGGGATCCTAAACCATGGCCCCCAGGCTCCTTTTC) and F5β-bottom (GGAAGGGGGCGGCCGCTCAGGAATTTTTTTTCTTGACCATGG) primers and ligated into the pMX vector. In order to diversify the CDR3 region of the F5 TCRβ chain, the F5β-CDR3-HM primer (CTGGTCCGAAGAACTGCTCAGCATGCCCCCCAGTCCGGGAGCTGCTTGCACAAAGATACA C) was synthesized, in which the CDR3 coding sequence contains 70% of the original nucleotide (underlined) and 10% of each of the other 3 nucleotides. A 5' fragment of the F5 TCRβ was amplified by PCR with F5β-top and F5β-CDR3-HM primers and a 3' fragment was amplified with F5β-CDR3-3'top (GAGCAGTTCTTCGGACCAG) and F5β-bottom primers. Both resulting F5 TCRβ fragments were assembled by PCR 14 in the presence of F5β-top and F5β-bottom primers and this TCRβ CDR3 DNA library was ligated into the pMX vector. Ligation products were introduced into *Escherichia coli* MC1061 cells by electroporation to generate a CDR3 library with a complexity of 3x10⁶ clones. **Flow cytometric analysis and TCR CDR3 library screening.** Aspecific staining to 34.1L cells was blocked with 0.5 µg/ml anti-FcgRII/III mAb (clone 2.4G2). Cells were stained with PE conjugated anti-TCRβ chain (H57-597) mAb (Pharmingen) or MHC tetramers at 4°C (unless indicated otherwise). Propidium iodide (1 µg/ml) (Sigma) was included prior to analysis. Data acquisition and analysis was performed on a FacsCalibur (Becton Dickinson, MountainView, CA) using CellQuest software. Cell sorting was performed on a FACStar Plus (Becton Dickinson, MountainView, CA) using Lysis II software.**34.1Lζ stimulation assay.** The SIN-(NFAT)₆-YFP retroviral construct was produced as described previously (Hooijberg *et al*, ms. submitted). TCR expressing 34.1Lζ cells were transduced with the self-inactivating retroviral construct. Transduced cells, as revealed by YFP expression after overnight PMA (10 µg/ml) (Sigma) and ionomycin (1.67 µg/ml) (Sigma) stimulation, were isolated by flow cytometry. Transduced 34.1Lζ cells were incubated overnight at 37°C with EL4 target cells at an effector:target ratio of 1:10 in the presence of peptides at the indicated concentrations. The percentage of YFP expressing 34.1Lζ cells was determined by flow cytometric analysis.

**Determination of MHC-TCR dissociation rates**. Cells were stained with (APC)-labeled H-2D^{b} tetramers for 20 minutes at 4°C, and subsequently washed once with PBS/0.5% BSA/0.02% NaN₃. Following addition of unlabeled homologous H-2D^{b} monomers (10 µM) the decay of tetramer staining was measured by flow cytometry. MHC/TCR dissociation was calculated as follows: (FIₑₓₚ - FI₀)/(FIₘₐₓ - FI₀) x 100%. Simultaneous addition of H-2D^{b} tetramers and 10 µM unlabeled homologous H-2D^{b} monomers during cell labeling completely prevents the binding of tetrameric MHC complexes (not shown).

### References

1. Sprent, J. & Kishimoto, H. T cell tolerance and the thymus. *Ann N Y Acad Sci* **841**, 236-245 (1998).
2. Stockinger, B. T lymphocyte tolerance: from thymic deletion to peripheral control mechanisms. *Adv Immunol* **71,** 229-265 (1999).
3. Clackson, T., Hoogenboom, H.R., Griffiths, A.D. & Winter, G. Making antibody fragments using phage display libraries. *Nature* **352**, 624-628 (1991).
4. Weidanz, J.A., Card, K.F., Edwards, A., Perlstein, E. & Wong, H.C. Display of functional alphabeta single-chain T-cell receptor molecules on the surface of bacteriophage. J *Immunol Methods* **221,** 59-76 (1998).
5. Monks, C.R., Freiberg, B.A., Kupfer, H., Sciaky, N. & Kupfer, A. Three-dimensional segregation of supramolecular activation clusters in T cells. *Nature* **395**, 82-86 (1998).
6. Grakoui, A. *et al.* The immunological synapse: a molecular machine controlling T cell activation. *Science* **285**, 221-227 (1999).
7. Garboczi, D.N. *et al.* Structure of the complex between human T-cell receptor, viral peptide and HLA-A2. *Nature* **384**, 134-141 (1996) .
8. Garcia, K.C. *et al.* An alphabeta T cell receptor structure at 2.5 A and its orientation in the TCR-MHC complex. *Science* **274**, 209-219 (1996).
9. Altman, J.D. *et al.* Phenotypic analysis of antigen-specific T lymphocytes. *Science* **274,** 94-96 (1996).
10. Moskophidis, D. & Kioussis, D. Contribution of virus-specific CD8+ cytotoxic T cells to virus clearance or pathologic manifestations of influenza virus infection in a T cell receptor transgenic mouse model. *J Exp Med* **188,** 223-232 (1998).
11. Townsend, A.R., Gotch, F.M. & Davey, J. Cytotoxic T cells recognize fragments of the influenza nucleoprotein. *Cell* **42**, 457-467 (1985).
12. Ding, Y.H. *et al.* Two human T cell receptors bind in a similar diagonal mode to the HLA- A2/Tax peptide complex using different TCR amino acids. *Immunity* **8**, 403-411 (1998).
13. Teng, M.K. *et al.* Identification of a common docking topology with substantial variation among different TCR-peptide-MHC complexes. *Curr Biol* **8,** 409-412 (1998).
14. Crameri, A., Cwirla, S. & Stemmer, W.P. Construction and evolution of antibody-phage libraries by DNA shuffling. *Nat Med* **2**, 100-102 (1996).
15. Fiering, S. *et al.* Single cell assay of a transcription factor reveals a threshold in transcription activated by signals emanating from the T-cell antigen receptor. *Genes Dev* **4**, 1823-1834 (1990).
16. Karttunen, J. & Shastri, N. Measurement of ligand-induced activation in single viable T cells using the lacZ reporter gene. *Proc Natl Acad Sci U S A* **88**, 3972-3976 (1991).
17. Kersh, G.J., Kersh, E.N., Fremont, D.H. & Allen, P.M. High- and low-potency ligands with similar affinities for the TCR: the importance of kinetics in TCR signaling. *Immunity* **9,** 817-826 (1998).
18. Busch, D.H. & Pamer, E.G. T cell affinity maturation by selective expansion during infection. *J Exp Med* **189,** 701-710 (1999).
19. Savage, P.A., Boniface, J.J. & Davis, M.M. A kinetic basis for T cell receptor repertoire selection during an immune response. *Immunity* **10**, 485-492 (1999).
20. Arstila, T.P. *et al.* A direct estimate of the human alphabeta T cell receptor diversity. *Science* **286**, 958-961 (1999).
21. Clay, T.M. *et al.* Efficient transfer of a tumor antigen-reactive TCR to human peripheral blood lymphocytes confers anti-tumor reactivity. *J Immunol* **163,** 507-513 (1999).
22. Kappler, J.W., Roehm, N. & Marrack, P. T cell tolerance by clonal elimination in the thymus. *Cell* **49,** 273-280 (1987).
23. Kisielow, P., Bluthmann, H., Staerz, U.D., Steinmetz, M. & von Boehmer, H. Tolerance in T-cell-receptor transgenic mice involves deletion of nonmature CD4+8+ thymocytes. *Nature* **333**, 742-746 (1988).
24. Sha, W.C. *et al.* Positive and negative selection of an antigen receptor on T cells in transgenic mice. *Nature* **336**, 73-76 (1988).
25. Pircher, H., Burki, K., Lang, R., Hengartner, H. & Zinkernagel, R.M. Tolerance induction in double specific T-cell receptor transgenic mice varies with antigen. *Nature* 342, 559-561 (1989).
26. Theobald, M. *et al.* Tolerance to p53 by A2.1-restricted cytotoxic T lymphocytes. *J Exp Med* **185,** 833-841 (1997).
27. Morgan, D.J. *et al.* Activation of low avidity CTL specific for a self epitope results in tumor rejection but not autoimmunity. *J Immunol* **160**, 643-651 (1998).
28. Romieu, R. *et al.* Passive but not active CD8+ T cell-based immunotherapy interferes with liver tumor progression in a transgenic mouse model. *J Immunol* **161**, 5133-5137 (1998).
29. Wang, R., Wang-Zhu, Y., Gabaglia, C.R., Kimachi, K. & Grey, H.M. The stimulation of low-affinity, nontolerized clones by heteroclitic antigen analogues causes the breaking of tolerance established to an immunodominant T cell epitope. *J Exp Med* **190**, 983-994 (1999).
30. Zeh, H.J., 3rd, Perry-Lalley, D., Dudley, M.E., Rosenberg, S.A. & Yang, J.C. High avidity CTLs for two self-antigens demonstrate superior in vitro and in vivo antitumor efficacy. *J Immunol* **162**, 989-994 (1999).
31. Haanen, J.B. *et al.* Systemic T cell expansion during localized viral infection. *Eur J Immunol* **29**, 1168-1174 (1999).
32. Oosterwegel, M., Timmerman, J., Leiden, J. & Clevers, H. Expression of GATA-3 during lymphocyte differentiation and mouse embryogenesis. *Dev Immunol* **3**, 1-11 (1992).
33. Gorer, P.A. Studies in antibody response of mice to tumour inoculation. *Br. J. Cancer* **4**, 372-379 (1950).
34. Hanenberg, H. *et al.* Colocalization of retrovirus and target cells on specific fibronectin fragments increases genetic transduction of mammalian cells. *Nat Med* **2**, 876-882 (1996).
35. Whelan, J.A. *et al.* Specificity of CTL interactions with peptide-MHC class I tetrameric complexes is temperature dependent. *J Immunol* **163**, 4342-4348 (1999).

### Legends to figures

Figure 1: Left: schematic representation of the generation and screening of retroviral TCR display libraries. Right: generation of the TCR library F5 TCR-1. Complementarity-determining regions of the TCRα and β chains are depicted as solid boxes. The complementarity-determining region 3 DNA sequence of the β chain targeted in the current experiments is depicted in bold.

Figure 2: MHC tetramer analysis of in vitro-selected TCRs. 2A. Flow cytometric analysis of 34.1Lζ cells expressing the F5 (top panels), NT-1 (middle panels), or PR-1 TCRs (bottom panels). Left panels represent staining with anti-TCR antibody. Middle panels represent staining with APC-labeled tetrameric H-2D^{b} complexes containing the A/NT/60/68 nucleoprotein epitope (ASNENMDAM), right panels represent staining with APC-labeled H-2D^{b} tetramers containing the A/PR8/34 nucleoprotein epitope (ASNENMETM). Tetramer staining was performed at 37°C ³⁵. 2B. Selection of influenza A-reactive TCRs from *in vitro* TCR libraries. Panels represent staining of the TCRβ CDR3 library with APC-labeled tetrameric H-2D^{b} complexes containing the A/NT/60/68 nucleoprotein epitope prior to screening (top panel) and after 1 (middle panel) and 2 (bottom panel) sorts with A/NT/60/68 H-2D^{b} tetramers.

Figure 3: Signaling function of *in vitro* selected TCRs. 34.1Lζ TCR-expressing cells transduced with the NFAT-YFP construct were exposed to EL4 target cells (E:T ratio 1:10) in the presence of different concentrations of either the A/NT/60/68 (open squares) or A/PR8/34 (filled circles ) T cell epitope. Sensitivity and specificity of the different TCRs were determined by flow cytometric analysis of the percentage of YFP expressing 34.1L cells. In accordance with previous results, the distribution of YFP expression upon stimulation is bimodal ^{15,16} and T cell activation upon stimulation with PMA and ionomycin results in 60-65% YFP expressing cells (not shown). Data shown are means of triplicates +/- S.D.

Figure 4: Specificity of the PR-1 TCR. Left: 34.1Lζ PR-1-expressing cells transduced with the NFAT-YFP construct were exposed to EL4 target cells or EL4^{PR} cells that endogenously produce the A/PR8/34 CTL epitope, at an E:T ratio of 1:10. Right: 34.1Lζ PR-1-expressing cells were incubated with cell suspensions from the indicated tissues at an E:T ratio of 1:100. In the left panel the percentage of YFP-positive cells in the absence of target cells is depicted. In the right panel the percentage of YFP-positive cells in the presence of spleen cells incubated with 0.5 *µ*M of the ASNENMETM peptide is depicted. Data shown are means of triplicates (left) or duplicates (right).

Figure 5: Determination of MHC-TCR dissociation rates. 34.1Lζ-TCR expressing cells were stained with their cognate APC-labeled peptide/H-2D^{b} tetramers at 4°C and subsequently exposed to an excess of homologous unlabeled H-2D^{b} monomers at 25°C. Decay of H-2D^{b} tetramer staining was measured by flow cytometry and is plotted as the percentage of maximum staining.

## Claims

1. A method for generating at least one receptor having a desired specificity and/or affinity for a ligand, whereby said receptor undergoes functional processing after ligand-binding, comprising constructing a sequence encoding such a receptor and allowing for the product of said sequence to be expressed in a suitable environment wherein said processing after ligand binding can occur.

2. A method according to claim 1, wherein said at least one receptor is a membrane associated receptor.

3. A method according to claim 1 or 2 wherein said receptor is a T cell receptor.

4. A method according to any one of claims 1-3, wherein said environment is a host cell.

5. A method according to claim4, wherein said host cell is a T cell receptor negative T cell.

6. A method according to any one of claims 4-5, wherein said constructed sequence is stably associated with the host cell.

7. A method according to any one of claims 1-6, wherein said constructed sequence is a sequence derived from a retrovirus.

8. A method to any one of claims 1-7, wherein said functional processing comprises clustering of at least two receptors.

9. A method according to any one of claims 1-8, wherein said functional processing comprises binding to other proteinaceous structures.

10. A method according to any one of claims 1-9, wherein said receptor's affinity is changed through a mutation in said constructed sequence.

11. A method according to claim 10, whereby said receptor's affinity is changed to an affinity and/or specificity normally not present in said receptor's natural surrounding.

12. A method according to claim 11, wherein said receptor is a T cell receptor having affinity for a self antigen, a tumour antigen and/or a pathogen derived antigen.

13. A method according to any one of claims 1-12 wherein a number of different constructed sequences are brought into separate suitable environments providing a library of environments having receptors with different ligand binding affinities.

14. A library of receptors having different ligand binding affinities obtainable by a method according to claim 13.

15. A library according to claim 14, wherein said receptors are T cell receptors.

16. A library according to claim 14 or 15, wherein said suitable environments are host cells.

17. A library according to claim 16, wherein said host cells are T cell receptor negative T cells.

18. A library according to any one of claims 14-17, wherein said constructed sequences comprise sequences derived from a retrovirus.

19. A method for selecting a T cell receptor or a sequence encoding the same, comprising contacting a ligand to be recognised by said T cell receptor with a library according to any one of claims 14-18 in the appropriate context and selecting at least one binding T cell receptor from said library.

20. A method according to claim 19, wherein said ligand is presented in the context of an appropriate MHC molecule.

21. A T cell receptor or a sequence encoding the same obtainable by a method according to claim 19 or 20.

22. A T cell receptor according to claim 21 having binding affinity for a tumour antigen and/or a self antigen.

23. A method for providing a T cell with the capability of binding a desired presented antigen, comprising providing said T cell with a T cell receptor or a sequence encoding it according to claim 21 or 22.

24. A T cell capable of binding a desired antigen obtainable by a method according to claim 23.

25. A method for providing a subject with additional capability of generating a response against antigens of undesired cells or pathogens, comprising providing said subject with at least one T cell according to claim 24.

26. A method according to claim 25 wherein said T cell is derived from said subject.

27. A method according to claim 25 wherein said T cell is HLA matched for said subject.
